# EUROPÄISCHE PATENTSCHRIFT

(11) **EP 3 243 436 B1**
(45) Veröffentlichungstag und Bekanntmachung des Hinweises auf die Patenterteilung: **27.05.2020**
(21) Anmeldenummer: 17164786.0
(22) Anmeldetag: 04.04.2017
(51) Int. Cl.: A61B 6/03, A61B 6/00, G06F 1/16

(54) **ANORDNUNG MIT EINER TABLETCOMPUTEREINHEIT UND EINER GANTRY EINER MEDIZINISCHEN BILDGEBUNGSVORRICHTUNG**
ASSEMBLY WITH A TABLET COMPUTER UNIT AND A GANTRY OF A MEDICAL IMAGING DEVICE
SYSTÈME COMPRENANT UNE TABLETTE TACTILE ET PORTIQUE MOBILE DE DISPOSITIF D'IMAGERIE MÉDICALE

(43) Veröffentlichungstag der Anmeldung: 15.11.2017
(73) Patentinhaber: Siemens Healthcare GmbH, 91052 Erlangen (DE)
(72) Erfinder: Müller, Hans-Jürgen, 91362 Pretzfeld (DE)

(56) Entgegenhaltungen:
- DE-A1-102013 226 342
- DE-A1-102014 226 756
- DE-A1-102015 211 148
- US-A1- 2014 275 953
- US-A1- 2016 246 328

## Beschreibung

Bisher wurden Computertomographiegeräte (CT-Geräte) in der Regel mit Hilfe von Bedienelementen bedient, welche fest an der Gantry angeordnet sind, insbesondere in einer Verkleidung der Gantry integriert sind. Medizinische Bildgebungsvorrichtungen können auch mittels eines Tabletcomputers bedient werden. Bei einer medizinischen Untersuchung, welche mit der medizinischen Bildgebungsvorrichtung durchgeführt wird, kann es vorteilhaft sein, wenn der Tabletcomputer für bestimmte Untersuchungsschritte an der Gantry angeordnet werden kann und für andere Untersuchungsschritte von der Gantry losgelöst benutzt werden kann.

Die DE 10 2014 226 287 A1 offenbart ein Verfahren zur Bedienung eines medizinischen Geräts mit einer Gantry, umfassend folgende Schritte:
- Darstellen einer graphischen Benutzeroberfläche zur Bedienung des medizinischen Geräts in einer ersten Ausgabeeinheit eines ersten berührungsempfindlichen Bildschirms,
- Eingeben von Bedieninformationen mittels der graphischen Benutzeroberfläche durch die erste Eingabeeinheit des ersten berührungsempfindlichen Bildschirms,
- wobei der erste berührungsempfindliche Bildschirm an der Gantry und/oder in der Nähe der Gantry angeordnet ist.

Die US 2015/0169001 A1 offenbart eine medizintechnische Anlage, mit einem bildgebenden medizintechnischen Gerät, sowie mit mindestens einer portablen Bedien-/Anzeigeeinheit, dadurch gekennzeichnet, dass das bildgebende medizintechnische Gerät eine Anzahl jeweils eine geschlossene Oberfläche aufweisender Andockstationen aufweist, welche dazu ausgebildet sind, eine drahtlose Datenverbindung mit der Bedien-/Anzeigeeinheit herzustellen und diese zugleich in definierter Position am bildgebenden medizintechnischen Gerät zu halten.

Die US 2015/0313562 A1 offenbart ein Verfahren zum Abrufen von Anwendungsbefehlen zu einer auf einer touchfähigen Benutzeroberfläche einer Recheneinheit eines medizinischen Bildgebungssystems dargestellten medizinischen Darstellung, zumindest umfassend die Schritte:
1.1. gleichzeitiges Berühren der Benutzeroberfläche an mindestens zwei Berührpunkten, und
1.2. Anzeigen der anwendungssituationsbedingt möglichen Anwendungsbefehle auf der Benutzeroberfläche an den Berührpunkten.

Die US 2016/0296197 A1 offenbart ein CT-System zur computertomographischen Untersuchung, insbesondere eines Patienten, zumindest aufweisend mindestens ein mobiles Bedienelement zur Steuerung des CT-Systems, welches drahtlos mit dem CT-System verbunden ist.

Die Erfindung hat die Aufgabe, eine verbesserte, insbesondere flexible, Anordnung einer Tabletcomputereinheit an einer Gantry einer medizinischen Bildgebungsvorrichtung zu ermöglichen.

Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft eine Anordnung, aufweisend eine Tabletcomputereinheit und eine Gantry einer medizinischen Bildgebungsvorrichtung,
- wobei die Tabletcomputereinheit einen Tabletcomputer und eine erste Verbindungseinheit aufweist,
- wobei die Gantry eine Verkleidung und eine zweite Verbindungseinheit aufweist,
- wobei die Verkleidung eine Vertiefung aufweist, in welche die Tabletcomputereinheit formschlüssig aufnehmbar, insbesondere aufgenommen, ist,
- wobei die zweite Verbindungseinheit in einem Bereich der Vertiefung derart angeordnet ist, dass mittels der ersten Verbindungseinheit und der zweiten Verbindungseinheit eine Verbindung gebildet werden kann, welche einem Entfernen der Tabletcomputereinheit aus der Vertiefung entgegenwirkt,
- wobei die Vertiefung einen ersten Bereich zur formschlüssigen Aufnahme des Tabletcomputers und einen zweiten Bereich zur Aufnahme der ersten Verbindungseinheit aufweist,
- wobei der zweite Bereich als zumindest eine Vertiefung relativ zu dem ersten Bereich ausgebildet ist,
- wobei die Vertiefung einen dritten Bereich aufweist, in dem die formschlüssige Aufnahme des Tabletcomputers derart unterbrochen ist, dass in der Vertiefung ein Finger und/oder ein Werkzeug an den Tabletcomputer angelegt werden kann, um eine Druckkraft zum Entfernen der Tabletcomputereinheit aus der Vertiefung auszuüben.

Insbesondere kann die zweite Verbindungseinheit in die Verkleidung integriert sein und/oder an der Verkleidung angeordnet sein. Insbesondere kann die zweite Verbindungseinheit von der Verkleidung verdeckt angeordnet sein. Insbesondere kann die erste Verbindungseinheit in den Tabletcomputer integriert sein und/oder an dem Tabletcomputer angeordnet sein.

Insbesondere kann die erste Verbindungseinheit zumindest einen metallischen Bereich und die zweite Verbindungseinheit zumindest einen Magneten aufweisen. Insbesondere kann die erste Verbindungseinheit zumindest einen Magneten und die zweite Verbindungseinheit zumindest einen metallischen Bereich aufweisen.

Insbesondere kann der Tabletcomputer ein Gehäuse mit einem metallischen Gehäusebereich aufweisen. Insbesondere kann der metallische Gehäusebereich die erste Verbindungseinheit bilden. Insbesondere kann der metallische Bereich und/oder der metallische Gehäusebereich ferromagnetisch sein. Insbesondere kann der Magnet ein Permanentmanget und/oder ein Elektromagnet sein.

Insbesondere kann die Vertiefung eine Adapterschale aufweisen, in welche die Tabletcomputereinheit formschlüssig aufnehmbar, insbesondere aufgenommen, ist.

Insbesondere kann die Tabletcomputereinheit ein erstes Energieübertragungselement zum Empfang elektrischer Energie für den Tabletcomputer aufweisen. Insbesondere kann die Gantry ein mit dem ersten Energieübertragungselement korrespondierendes zweites Energieübertragungselement zum Bereitstellen der elektrischen Energie für den Tabletcomputer aufweisen.

Insbesondere kann die erste Verbindungseinheit mehrere, beispielsweise zwei, Metallplatten aufweisen. Insbesondere kann die zweite Verbindungseinheit mehrere, beispielsweise zwei, Magnete aufweisen, welche mit den mehreren Metallplatten korrespondierend angeordnet sind. Gemäß einer Ausführungsform der Erfindung ist vorgesehen, dass das erste Energieübertragungselement zwischen den zwei Metallplatten angeordnet ist und/oder dass das zweite Energieübertragungselement zwischen den zwei Magneten angeordnet ist.

Insbesondere kann das erste Energieübertragungselement zumindest ein erstes induktives Element aufweisen. Insbesondere kann das zweite Energieübertragungselement zumindest ein zweites induktives Element aufweist. Unter einem induktiven Element kann insbesondere eine Spule verstanden werden.

Insbesondere kann die Anordnung ferner eine Befestigungsschale aufweisen, in welche die Tabletcomputereinheit formschlüssig aufnehmbar, insbesondere aufgenommen, ist,
- wobei die Befestigungsschale unabhängig von der Gantry an einer Tragkonstruktion anordenbar ist,
- wobei die Befestigungsschale eine dritte Verbindungseinheit aufweist, welche derart angeordnet ist, dass mittels der ersten Verbindungseinheit und der dritten Verbindungseinheit eine insbesondere magnetische Verbindung gebildet werden kann, welche einem Entfernen der Tabletcomputereinheit aus der Befestigungsschale entgegenwirkt.

Insbesondere kann die Anordnung ferner die medizinische Bildgebungsvorrichtung aufweisen. Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem PET-CT-Gerät und einem PET-MR-Gerät, besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Ohne Einschränkung des allgemeinen Erfindungsgedankens wird bei einigen der Ausführungsformen ein Computertomographiegerät beispielhaft für eine medizinische Bildgebungsvorrichtung genannt.

Gemäß einer Ausführungsform der Erfindung weist die medizinische Bildgebungsvorrichtung eine Akquisitionseinheit, welche zur Akquisition der Akquisitionsdaten ausgebildet ist, auf. Insbesondere kann die Akquisitionseinheit eine Strahlungsquelle und einen Strahlungsdetektor aufweisen. Eine Ausführungsform der Erfindung sieht vor, dass die Strahlungsquelle zur Emission und/oder zur Anregung einer Strahlung, insbesondere einer elektromagnetischen Strahlung, ausgebildet ist und/oder dass der Strahlungsdetektor zur Detektion der Strahlung, insbesondere der elektromagnetischen Strahlung, ausgebildet ist. Die Strahlung kann beispielsweise von der Strahlungsquelle zu einem abzubildenden Bereich gelangen und/oder nach einer Wechselwirkung mit dem abzubildenden Bereich zu dem Strahlungsdetektor gelangen. Bei der Wechselwirkung mit dem abzubildenden Bereich wird die Strahlung modifiziert und damit zum Träger von Informationen, die den abzubildenden Bereich betreffen. Bei der Wechselwirkung der Strahlung mit dem Detektor werden diese Informationen in Form von Akquisitionsdaten erfasst.

Insbesondere bei einem Computertomographiegerät und bei einem C-Bogen-Röntgengerät können die Akquisitionsdaten Projektionsdaten, die Akquisitionseinheit eine Projektionsdaten-Akquisitionseinheit, die Strahlungsquelle eine Röntgenquelle, der Strahlungsdetektor ein Röntgendetektor sein. Der Röntgendetektor kann insbesondere ein quantenzählender und/oder energieauflösender Röntgendetektor sein.

Insbesondere bei einem Magnetresonanztomographiegerät können die Akquisitionsdaten ein Magnetresonanzdatensatz, die Akquisitionseinheit eine Magnetresonanzdaten-Akquisitionseinheit, die Strahlungsquelle eine erste Hochfrequenzantenneneinheit, der Strahlungsdetektor die erste Hochfrequenzantenneneinheit und/oder eine zweite Hochfrequenzantenneneinheit sein.

Die Gantry einer medizinischen Bildgebungsvorrichtung weist typischerweise eine Tragkonstruktion auf, an der insbesondere Komponenten der Akquisitionseinheit, insbesondere die Strahlungsquelle und/oder der Strahlungsdetektor, angeordnet sind. Die Tragkonstruktion der Gantry weist typischerweise eine derart hohe Steifigkeit und Festigkeit auf, dass die Komponenten der Akquisitionseinheit sowohl relativ zueinander als auch relativ zu einem abzubildenden Bereich in einer für die Bildgebung hinreichend definierten Geometrie anordenbar sind. Bei einem Computertomographiegerät weist die Gantry typischerweise einen Tragrahmen und einen relativ zu dem Tragrahmen drehbar gelagerten Rotor auf, wobei die Strahlungsquelle und der Strahlungsdetektor an dem Rotor angeordnet sind. Optional kann die Gantry einen relativ zu dem Tragrahmen kippbar gelagerten Kipprahmen aufweisen, wobei der Rotor an dem Kipprahmen angeordnet ist.

Bei einem C-Bogen-Röntgengerät weist die Gantry typischerweise einen Tragrahmen und einen relativ zu dem Tragrahmen schwenkbar gelagerten C-Bogen auf, wobei die Strahlungsquelle und der Strahlungsdetektor an dem C-Bogen angeordnet sind.

Bei einem Magnetresonanztomographiegerät weist die Gantry typischerweise einen Tragrahmen auf, an dem der Hauptmagnet und eine erste Hochfrequenzantenneneinheit angeordnet sind, wobei die erste Hochfrequenzantenneneinheit in Form einer Körperspule, die dem Fachmann auch unter dem Begriff "Body Coil" bekannt ist, ausgebildet ist.

Die erfindungsgemäße Lösung ermöglicht insbesondere ein einfaches und intuitives Anordnen der Tabletcomputereinheit an der Gantry und Lösen der Tabletcomputereinheit von der Gantry realisierbar.

Insbesondere kann vorgesehen sein, dass die Tabletcomputereinheit derart formschlüssig in die Vertiefung aufnehmbar, insbesondere aufgenommen, ist, dass eine Flächennormale des Bildschirms des Tabletcomputers im Wesentlichen senkrecht, insbesondere senkrecht, zu einer Oberfläche der Verkleidung ist.

Die Oberfläche der Verkleidung kann sich insbesondere in einem Randbereich der Verkleidung befinden, welcher an die Vertiefung angrenzt und/oder welcher die Vertiefung umgibt. Insbesondere kann vorgesehen sein, dass die Tabletcomputereinheit in Richtung einer Flächennormalen des Bildschirms in die Vertiefung einsetzbar und/oder aus der Vertiefung entfernbar ist.

Insbesondere kann die Vertiefung einen flächigen Auflagebereich aufweisen. Insbesondere kann vorgesehen sein, dass die Tabletcomputereinheit derart formschlüssig in die Vertiefung aufnehmbar, insbesondere aufgenommen, ist, dass der Tabletcomputer flächig auf dem flächigen Auflagebereich der Vertiefung aufliegt. Insbesondere kann vorgesehen sein, dass die Tabletcomputereinheit derart formschlüssig in die Vertiefung aufnehmbar, insbesondere aufgenommen, ist, dass der Tabletcomputer von der Verkleidung formschlüssig eingefasst ist.

Insbesondere kann die Verkleidung im Bereich der Vertiefung eine Oberfläche aufweisen, welche kantenlos und/oder glatt ausgebildet ist. Insbesondere kann die Verkleidung im Bereich der Vertiefung eine Oberfläche aufweisen, welche leicht zu reinigen ist. Damit können die Hygieneeigenschaften im Bereich der Vertiefung verbessert werden.

Insbesondere kann die Oberfläche der Verkleidung im Bereich der Vertiefung im Wesentlichen geschlossen, insbesondere geschlossen, sein. Dadurch kann eine Beeinträchtigung der Steifheit und/oder der Stabilität der Verkleidung der Gantry vermieden werden. Eine solche Beeinträchtigung kann beispielsweise durch eine Öffnung in der Verkleidung verursacht werden. Eine solche Öffnung kann beispielsweise zum Durchstecken und/oder Einfassen eines Rahmens, in dem ein Tabletcomputer angeordnet ist, vorgesehen sein.

Insbesondere kann vorgesehen sein, dass die Verbindung, welche mittels der ersten Verbindungseinheit und der zweiten Verbindungseinheit gebildet werden kann und welche einem Entfernen der Tabletcomputereinheit aus der Vertiefung entgegenwirkt, eine magnetische Verbindung ist. Insbesondere können sich die erste Verbindungseinheit und die zweite Verbindungseinheit gegenseitig magnetisch anziehen. Unter einem metallischen Bereich kann insbesondere eine Metallplatte verstanden werden.

Die Tabletcomputereinheit kann insbesondere ein Identifikationselement aufweisen, welches zur Identifikation der Tabletcomputereinheit ausgebildet ist. Gemäß einer Ausführungsform der Erfindung ist vorgesehen, dass das Identifikationselement von einem Benutzer visuell erfassbar ist und/oder dass das Identifikationselement nicht zerstörungsfrei von dem Tabletcomputer entfernbar ist. Das Identifikationselement kann insbesondere eine Gravur und/oder eine Bedruckung aufweisen.

Die Tabletcomputereinheit kann beispielsweise eine Metallplatte mit einer Gravur aufweisen. Insbesondere kann die erste Verbindungseinheit die Metallplatte mit der Gravur aufweisen. Die Metallplatte mit der Gravur kann insbesondere ferromagnetisch sein und/oder ein Verbindungspartner in der magnetischen Verbindung sein, welche einem Entfernen der Tabletcomputereinheit aus der Vertiefung entgegenwirkt. Mittels der Gravur ist beispielsweise eine insbesondere eindeutige Identifikation und/oder Zuordnung des Tabletcomputers realisierbar. Die Metallplatte mit der Gravur bildet somit das Identifikationselement. Beispielsweise kann der Tabletcomputer mittels des Identifikationselements, insbesondere mittels der Gravur, einer medizinischen Bildgebungsvorrichtung und/oder einem Untersuchungsraum zugeordnet werden.

Beispielsweise kann die Adapterschale lösbar mit der Verkleidung der Gantry verbindbar, insbesondere verbunden, sein. Die Adapterschalte kann insbesondere den zweiten Bereich zur Aufnahme der ersten Verbindungseinheit aufweisen. Die Adapterschale kann insbesondere zumindest eine Aussparung und/oder zumindest eine Vertiefung aufweisen, welche korrespondierend zu dem zumindest einen metallischen Bereich angeordnet ist. Mit Hilfe von Adapterschalen, deren Form jeweils an ein bestimmtes Modell eines Tabletcomputers angepasst ist, kann die Anordnung flexibel an verschiedene Modelle von Tabletcomputern angepasst werden.
Insbesondere kann das zweite Energieübertragungselement in dem Bereich der Vertiefung derart angeordnet sein, dass mittels des ersten Energieübertragungselements und des zweiten Energieübertragungselements eine Verbindung zur Übertragung der elektrischer Energie für den Tabletcomputer gebildet werden kann, wenn die Tabletcomputereinheit formschlüssig in die Vertiefung aufgenommen ist. Damit ist ein kabelloses, insbesondere induktives, Laden des Tabletcomputers möglich, während die Tabletcomputereinheit formschlüssig in die Vertiefung aufgenommen ist. Gegenüber einer Lösung, bei welcher eine lösbare Steckerverbindung an der Gantry zur elektrischen Energieversorgung des Tabletcomputers verwendet wird, ergeben sich insbesondere Vorteile in Bezug auf die Hygiene, die mechanische Beanspruchung und die Schnelligkeit, mit der die Verbindung hergestellt werden kann.

Die Befestigungsschale kann insbesondere als Wandhalterung für die Tabletcomputereinheit ausgebildet sein. Die Tabletcomputereinheit kann somit von der Gantry gelöst werden und beispielsweise in die Befestigungsschale aufgenommen werden. Daneben kann die Tabletcomputereinheit beispielsweise auf eine Ablage, beispielsweise einen Tisch, gelegt und auf der Ablage liegend bedient werden. Außerdem kann die Tabletcomputereinheit beispielsweise von einem Benutzer bedient werden, während der Benutzer die Tabletcomputereinheit in den Händen hält. Mit Merkmalen, die im Zusammenhang mit der Befestigungsschale beschrieben sind, kann auch die Gantry weitergebildet werden. Umgekehrt kann auch die Befestigungsschale mit Merkmalen weitergebildet werden, die im Zusammenhang mit der Gantry beschrieben sind.

Gemäß einer Ausführungsform der Erfindung weist die Tabletcomputereinheit keinen Rahmen auf, in welchen der Tabletcomputer aufnehmbar, insbesondere aufgenommen, ist und/oder mit welchem der Tabletcomputer lösbar verbindbar, insbesondere verbunden, ist. Gemäß einer Ausführungsform der Erfindung besteht die Tabletcomputereinheit aus dem Tabletcomputer und der ersten Verbindungseinheit. Gemäß einer Ausführungsform der Erfindung besteht die Tabletcomputereinheit aus dem Tabletcomputer und der ersten Verbindungseinheit und dem ersten Energieübertragungselement.

Gegenüber der Verwendung eines Tabletcomputers, welcher in einem Rahmen angeordnet ist, wobei der Rahmen korrespondierend zu einer für den Rahmen vorgesehenen Halterung an der Gantry angepasst ist, sind dadurch eine Reihe von Vorteilen realisierbar. Beispielsweise ergeben sich Vorteile in Bezug auf die Hygiene, insbesondere die Reinigung. Eine Dichtung zwischen dem Tabletcomputer und dem Rahmen muss einwandfrei passen, da ansonsten Flüssigkeit zwischen Panel und Rahmen eindringen kann. Die Reinigung ist in einem solchen Fall relativ aufwendig, da der Rahmen hierfür geöffnet und von dem Tabletcomputer separiert werden muss.

Gegenüber einer Lösung, bei welcher der Rahmen an der Gantry mittels eines Nut-Feder-Systems befestigt wird, ergibt sich insbesondere bei der magnetischen Verbindung ein Vorteil in Bezug auf die Hygiene, da das Nut-Feder-System typischerweise eine hygienische Schwachstelle ist. Der Tabletcomputer kann insbesondere zum Bedienen der medizinischen Bildgebungsvorrichtung ausgebildet sein. Unter einem Tabletcomputer kann im Kontext dieser Anmeldung auch ein Panel PC, welches zur kabellosen mobilen Benutzung ausgebildet ist, verstanden werden.

Die Erfindung ermöglicht somit insbesondere ein einfaches, intuitives und flexibles Anordnen der Tabletcomputereinheit an der Gantry sowie Lösen der Tabletcomputereinheit von der Gantry.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Die Verwendung von Ordnungszahlwörtern (erste, zweite, dritte etc.) in der Bezeichnung von Merkmalen dient im Kontext der vorliegenden Anmeldung vor allem der besseren Unterscheidbarkeit der unter Verwendung von Ordnungszahlwörtern bezeichneten Merkmale. Das Nichtvorhandensein eines Merkmals, welches durch eine Kombination eines gegebenen Ordnungszahlworts und eines Begriffs bezeichnet wird, schließt nicht aus, dass ein Merkmal vorhanden sein kann, welches durch eine Kombination eines dem gegebenen Ordnungszahlwort nachfolgenden Ordnungszahlworts und des Begriffs bezeichnet wird.

Im Folgenden werden ausgewählte Ausführungsformen der Erfindung unter Hinweis auf die beigefügten Figuren erläutert. Die Darstellung in den Figuren ist schematisch, stark vereinfacht und nicht zwingend maßstabsgetreu.

Es zeigen:
Fig. 1 eine schematische Darstellung einer beispielhaften Tabletcomputereinheit in einer ersten Ansicht,
Fig. 2 eine schematische Darstellung der beispielhaften Tabletcomputereinheit in einer zweiten Ansicht,
Fig. 3 eine schematische Darstellung einer beispielhaften Tabletcomputereinheit,
Fig. 4 eine schematische Darstellung eines Abschnitts einer beispielhaften Verkleidung einer Gantry,
Fig. 5 eine schematische Darstellung einer beispielhaften Anordnung,
Fig. 6 eine schematische Darstellung eines Abschnitts einer beispielhaften Verkleidung einer Gantry,
Fig. 7 eine schematische Darstellung einer beispielhaften Anordnung gemäß einer Ausführungsform der Erfindung,
Fig. 8 eine schematische Darstellung der beispielhaften Anordnung entlang einer Schnittlinie,
Fig. 9 eine schematische Darstellung einer beispielhaften Anordnung mit einer Adapterschale gemäß einer Ausführungsform der Erfindung,
Fig. 10 eine schematische Darstellung einer beispielhaften Anordnung mit einer Adapterschale gemäß einer Ausführungsform der Erfindung,
Fig. 11 eine schematische Darstellung einer Befestigungsschale in einer ersten Ansicht,
Fig. 12 eine schematische Darstellung der Befestigungsschale in einer zweiten Ansicht,
Fig. 13 eine schematische Darstellung der Befestigungsschale entlang einer Schnittlinie,
Fig. 14 eine schematische Darstellung einer beispielhaften Anordnung, und
Fig. 15 eine schematische Darstellung einer Anordnung mit einer medizinischen Bildgebungsvorrichtung gemäß einer Ausführungsform der Erfindung.

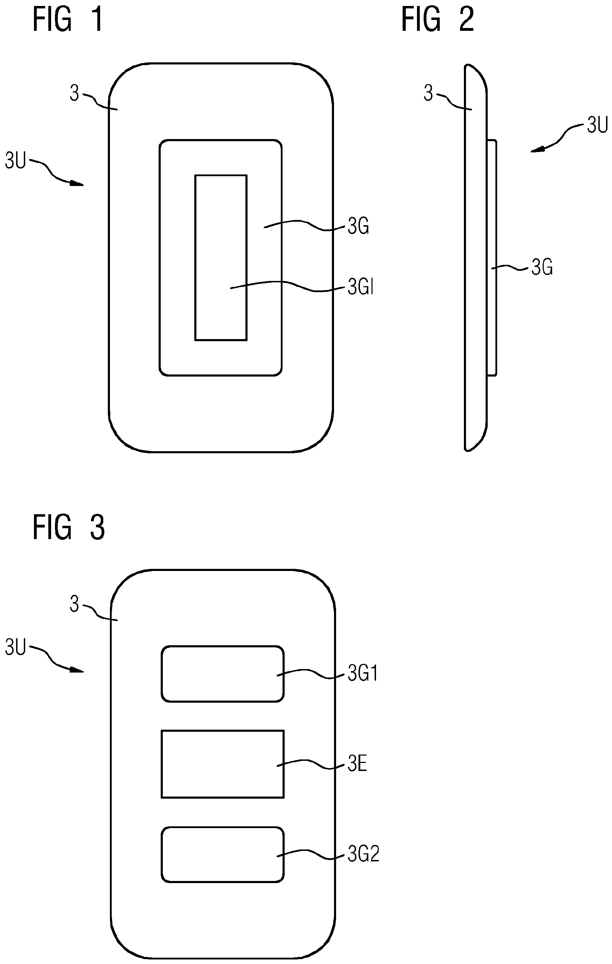

Fig. 1 eine schematische Darstellung einer beispielhaften Tabletcomputereinheit 3U in einer ersten Ansicht. Die Tabletcomputereinheit 3U weist einen Tabletcomputer 3 und eine erste Verbindungseinheit 3G auf. Die erste Verbindungseinheit 3G ist eine ferromagnetische Metallplatte mit einer Gravur 3GI. Fig. 2 zeigt die in der Fig. 1 dargestellte Tabletcomputereinheit in einer zweiten Ansicht, welche senkrecht zu der ersten Ansicht ist.

Fig. 3 zeigt eine schematische Darstellung einer beispielhaften Tabletcomputereinheit, wobei die Tabletcomputereinheit eine erste Verbindungseinheit aufweist, welche eine erste Metallplatte 3G1 und eine zweite Metallplatte 3G2 aufweist. Die Tabletcomputereinheit weist ferner ein erstes Energieübertragungselement 3E zum Empfang elektrischer Energie für den Tabletcomputer auf.

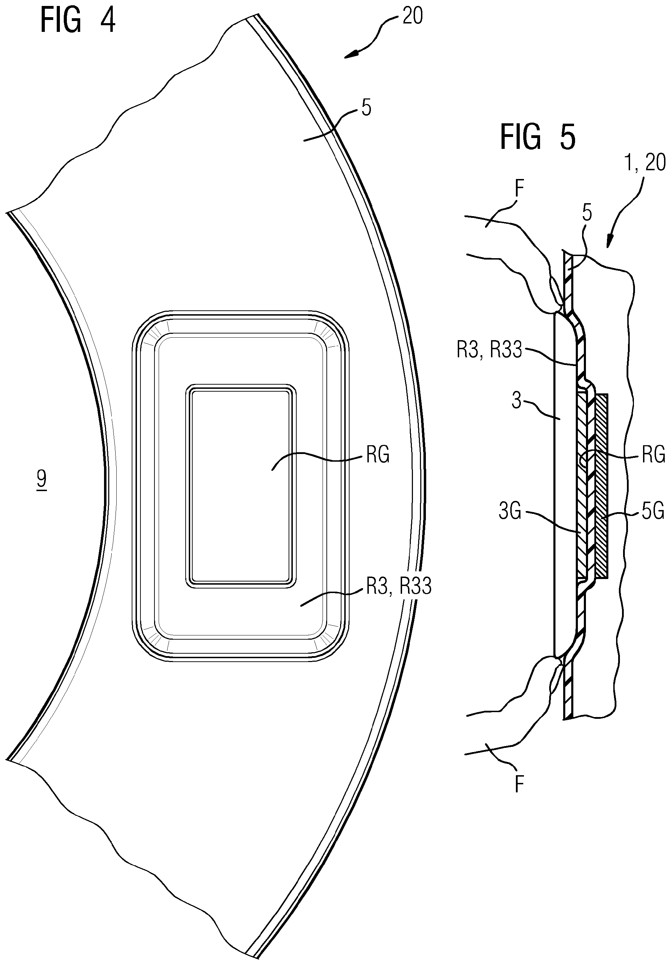

Fig. 4 zeigt eine schematische Darstellung eines Abschnitts einer beispielhaften Verkleidung 5 einer Gantry 20 einer medizinischen Bildgebungsvorrichtung. Die Verkleidung 5 weist eine Vertiefung R3 auf, in welche die Tabletcomputereinheit formschlüssig aufnehmbar ist. Die Vertiefung R3 weist einen ersten Bereich R33 zur formschlüssigen Aufnahme des Tabletcomputers 3 und einen zweiten Bereich RG zur Aufnahme der ersten Verbindungseinheit 3G auf.

Fig. 5 zeigt eine schematische Darstellung einer beispielhaften Anordnung 1. Die Anordnung 1 weist die in der Fig. 1 dargestellte Tabletcomputereinheit auf. Die Anordnung 1 weist ferner die Gantry 20 mit der in der Fig. 4 dargestellten Verkleidung 5 und einer zweiten Verbindungseinheit 5G auf. Die zweite Verbindungseinheit 5G ist ein Magnet, beispielsweise ein Permanentmagnet.

Die zweite Verbindungseinheit 5G ist in einem Bereich der Vertiefung derart angeordnet, dass mittels der ersten Verbindungseinheit 3G und der zweiten Verbindungseinheit 5G eine Verbindung, insbesondere eine magnetische Verbindung, gebildet werden kann, welche einem Entfernen der Tabletcomputereinheit aus der Vertiefung entgegenwirkt. Zwischen der ersten Verbindungseinheit 3G und der zweiten Verbindungseinheit 5G wirkt eine magnetische Anziehungskraft. Ein Randbereich des Tabletcomputers 3, der in der Vertiefung formschlüssig aufgenommen ist, ragt über einen Randbereich der Verkleidung, welcher an die Vertiefung angrenzt und welcher die Vertiefung umgibt, derart hinaus, dass an den Randbereich des Tabletcomputers ein Finger F angelegt werden, um eine Druckkraft zum Entfernen der Tabletcomputereinheit aus der Vertiefung auszuüben.

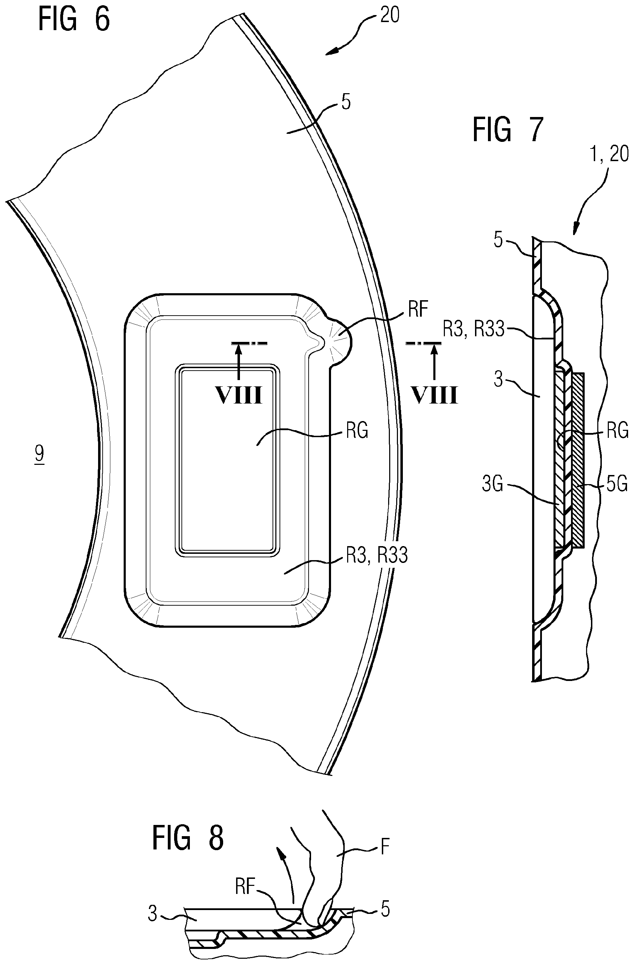

Fig. 6 zeigt eine schematische Darstellung eines Abschnitts einer beispielhaften Verkleidung einer Gantry, wobei die Vertiefung einen dritten Bereich RF aufweist, in dem die formschlüssige Aufnahme des Tabletcomputers derart unterbrochen ist, dass in der Vertiefung der Finger F an den Tabletcomputer angelegt werden kann, um eine Druckkraft zum Entfernen der Tabletcomputereinheit aus der Vertiefung auszuüben.

Fig. 7 zeigt eine schematische Darstellung einer beispielhaften Anordnung gemäß einer Ausführungsform der Erfindung, wobei die Anordnung 1 die Gantry 20 mit der in der Fig. 6 dargestellten Verkleidung 5 aufweist. Die Vertiefung ist derart ausgebildet, dass sich ein Bildschirm des Tabletcomputers, der in der Vertiefung formschlüssig aufgenommen ist, an die Oberfläche des Randbereichs der Verkleidung, welcher an die Vertiefung angrenzt und welcher die Vertiefung umgibt, bündig anschließt.

Fig. 8 zeigt eine schematische Darstellung der beispielhaften Anordnung entlang der Schnittlinie VIII-VIII in Fig. 6. Mit dem Finger F kann eine Druckkraft auf den Tabletcomputer 3 zum Entfernen der Tabletcomputereinheit aus der Vertiefung ausgeübt werden.

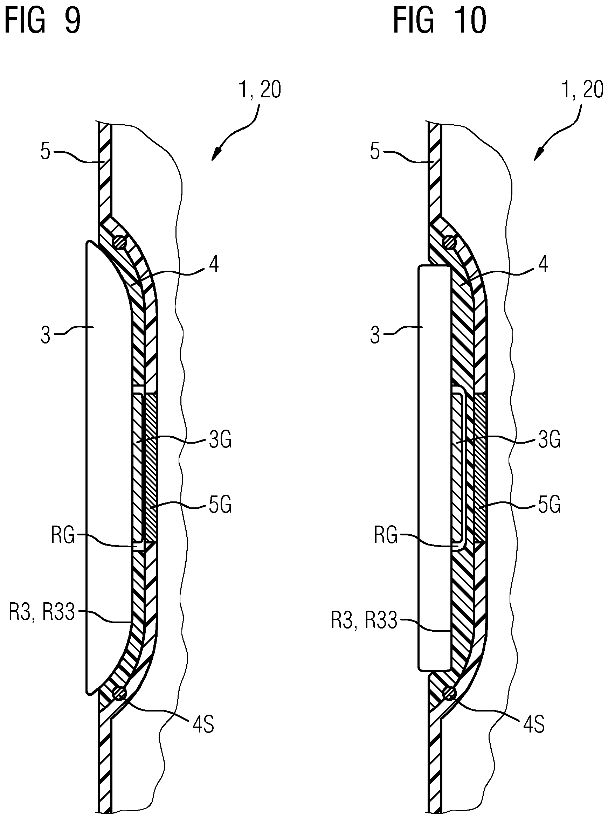

Fig. 9 zeigt eine schematische Darstellung einer beispielhaften Anordnung mit einer Adapterschale gemäß einer Ausführungsform der Erfindung. Die Tabletcomputereinheit ist formschlüssig in die Adapterschale 4 aufnehmbar, insbesondere aufgenommen. Die Adapterschale 4 weist eine Aussparung auf, in welche die erste Verbindungseinheit 3G aufnehmbar, insbesondere aufgenommen, ist. Die zweite Verbindungseinheit 5G ist in die Verkleidung 5 integriert.

Fig. 10 zeigt eine schematische Darstellung einer beispielhaften Anordnung mit einer Adapterschale gemäß einer Ausführungsform der Erfindung. Die Adapterschale 4 weist eine Vertiefung auf, in welche die erste Verbindungseinheit 3G aufnehmbar ist. Die Adapterschale 4 ist an die Form des jeweiligen Tabletcomputers 3 angepasst. Damit kann die Anordnung 1 an unterschiedliche Modelle von Tabletcomputern angepasst werden.

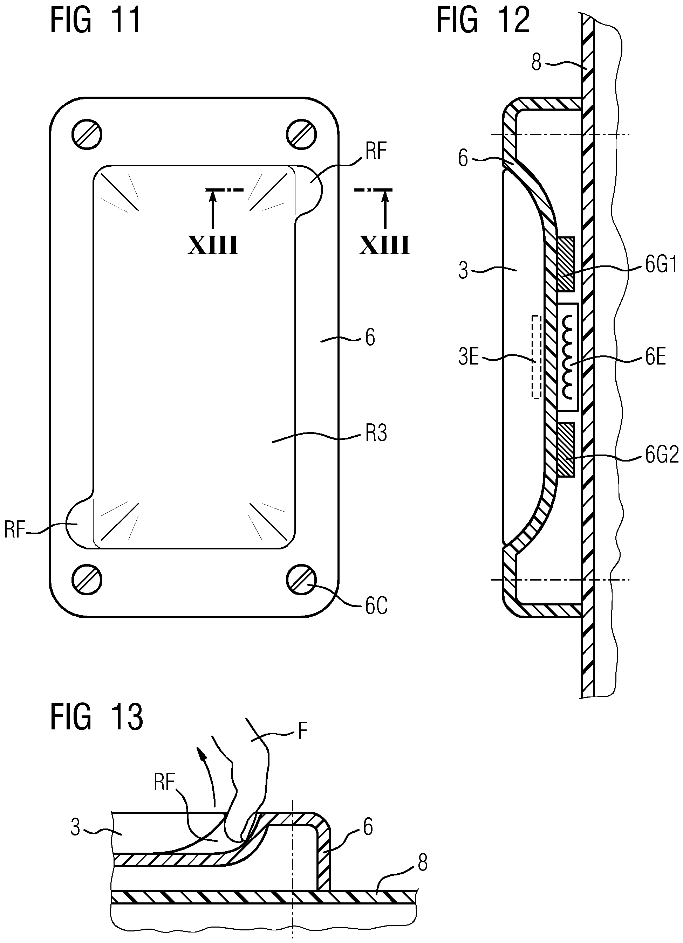

Fig. 11 zeigt eine schematische Darstellung einer Befestigungsschale 6 in einer ersten Ansicht. Die Befestigungsschale 6 kann beispielsweise mittels der Schrauben 6C an einer Wand befestigt werden. Die Befestigungungsschale 6 weist eine Vertiefung R3 auf, in welche die Tabletcomputereinheit formschlüssig aufnehmbar ist.

Fig. 12 zeigt eine schematische Darstellung der Befestigungsschale 6 in einer zweiten Ansicht. Die Befestigungsschale 6 ist an der Wand 8 befestigt. Die Befestigungsschale 6 weist eine dritte Verbindungseinheit auf, welche von zwei Magneten 6G1, 6G2 gebildet wird. Die Befestigungsschale weist ferner ein induktives Element 8E in Form einer Ladespule auf, welches zwischen den beiden Magneten 6G1 und 6G2 angeordnet ist. Der Tabletcomputer 3 weist ein metallisches Gehäuse auf, welches von den Magneten 6G1, 6G2 angezogen wird. Das metallische Gehäuse bildet somit die erste Verbindungseinheit. Der Tabletcomputer 3 weist ferner ein induktives Element 3E in Form einer Ladespule auf, welches korrespondierend mit dem induktiven Element 8E angeordnet ist. Mittels der beiden induktiven Elemente 3E, 8E kann elektrische Energie an den Tabletcomputer 3 übertragen werden, um beispielsweise einen Ackumulator des Tabletcomputers aufzuladen.
Fig. 13 zeigt eine schematische Darstellung der Befestigungsschale entlang einer Schnittlinie XIII-XIII in Fig. 11. Mit dem Finger F kann eine Druckkraft auf den Tabletcomputer 3 zum Entfernen der Tabletcomputereinheit aus der Befestigungsschale ausgeübt werden.

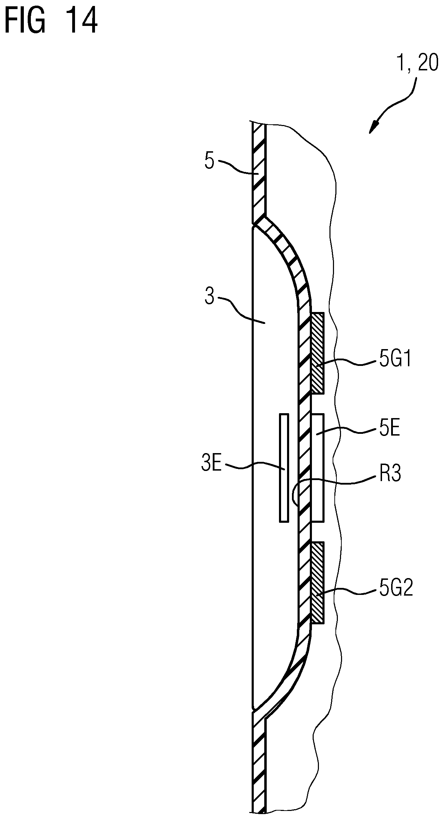

Fig. 14 zeigt eine schematische Darstellung einer beispielhaften Anordnung 1. Der Tabletcomputer 3 weist ein metallisches Gehäuse auf, welches von den Magneten 5G1, 5G2 angezogen wird. Das metallische Gehäuse bildet somit die erste Verbindungseinheit.

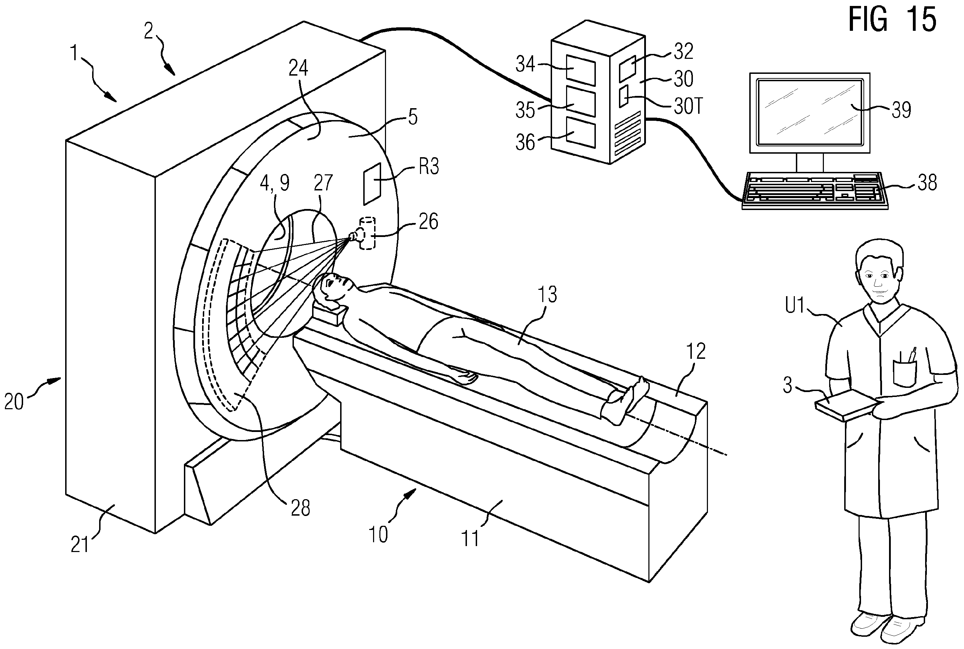

Fig. 15 zeigt eine schematische Darstellung einer Anordnung mit einer medizinischen Bildgebungsvorrichtung gemäß einer Ausführungsform der Erfindung. Ohne Beschränkung des allgemeinen Erfindungsgedankens ist für die medizinische Bildgebungsvorrichtung 2 beispielhaft ein Computertomographiegerät gezeigt.

Die medizinische Bildgebungsvorrichtung weist die Gantry 20 mit der Verkleidung 5 auf. Die Verkleidung 5 weist die Vertiefung R3 auf, in welche die Tabletcomputereinheit formschlüssig aufnehmbar ist. Die medizinische Bildgebungsvorrichtung 2 weist ferner die tunnelförmige Öffnung 9, die Patientenlagerungsvorrichtung 10 und die Steuerungsvorrichtung 30 auf.

Die Gantry 20 weist den stationären Tragrahmen 21 und den Rotor 24 auf. Der Rotor 24 ist mittels einer Drehlagerungsvorrichtung um eine Rotationsachse relativ zu dem stationären Tragrahmen drehbar angeordnet. In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.
Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12, insbesondere entlang der Systemachse AR, in den Akquisitionsbereich 4 einführbar ist.

Die medizinische Bildgebungsvorrichtung 2 ist zur Akquisition von Akquisitionsdaten basierend auf einer elektromagnetischen Strahlung 27 ausgebildet. Die medizinische Bildgebungsvorrichtung 2 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor.

Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 2 ausgebildet. Die Steuerungsvorrichtung 30 weist die Datenübertragungseinheit 30T, das computerlesbare Medium 32 und das Prozessorsystem 36 auf. Die Steuerungsvorrichtung 30 wird von einem Datenverarbeitungssystem, welches einen Computer aufweist, gebildet. Mittels der Datenübertragungseinheit 30T können Daten, insbesondere zur Bedienung der medizinischen Bildgebungsvorrichtung, zwischen dem Tabletcomputer 3 und der medizinischen Bildgebungsvorrichtung 2 kabellos übertragen werden. Der Tabletcomputer 3 weist eine Datenübertragungsschnittstelle, insbesondere zur kabellosen Datenübertragung, auf.

Die Anordnung 1 weist ferner die Energiebereitstellungseinheit 35 auf. Mittels der Energiebereitstellungseinheit 35 kann elektrische Energie an das zweite Energieübertragungselement bereitgestellt 5E werden. Die Energiebereitstellungseinheit 35 kann beispielsweise ein Anschluss an ein elektrisches Versorgungsnetzwerk sein.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden. Die medizinische Bildgebungsvorrichtung 2 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet. Alternativ oder zusätzlich können die Steuerungs-Information mittels des Tabletcomputers 3 eingegeben und/oder ausgegeben werden.

## Patentansprüche

1. Anordnung (1), aufweisend
- eine Tabletcomputereinheit (3U) mit einem Tabletcomputer (3) und einer ersten Verbindungseinheit (3G),
- eine Gantry (20) einer medizinischen Bildgebungsvorrichtung (2) mit einer Verkleidung (5) und einer zweiten Verbindungseinheit (5G),
- wobei die Verkleidung (5) eine Vertiefung (R3) aufweist, in welche die Tabletcomputereinheit (3U) formschlüssig aufnehmbar ist,
- wobei die zweite Verbindungseinheit (5G) in einem Bereich der Vertiefung (R3) derart angeordnet ist, dass mittels der ersten Verbindungseinheit (3G) und der zweiten Verbindungseinheit (5G) eine Verbindung gebildet werden kann, welche einem Entfernen der Tabletcomputereinheit (3U) aus der Vertiefung (R3) entgegenwirkt,
**dadurch gekennzeichnet, dass** die Vertiefung (R3) einen ersten Bereich (R33) zur formschlüssigen Aufnahme des Tabletcomputers (3) und einen zweiten Bereich (RG) zur Aufnahme der ersten Verbindungseinheit (3G) aufweist,
- wobei der zweite Bereich (RG) als zumindest eine Vertiefung relativ zu dem ersten Bereich (R33) ausgebildet ist,
- wobei die Vertiefung (R3) einen dritten Bereich (RF) aufweist, in dem die formschlüssige Aufnahme des Tabletcomputers (3) derart unterbrochen ist, dass in der Vertiefung ein Finger (F) und/oder ein Werkzeug an den Tabletcomputer (3) angelegt werden kann, um eine Druckkraft zum Entfernen der Tabletcomputereinheit (3U) aus der Vertiefung (R3) auszuüben.

2. Anordnung (1) nach Anspruch 1,
- wobei die zweite Verbindungseinheit (5G) in die Verkleidung (5) integriert ist und/oder an der Verkleidung (5) angeordnet ist.

3. Anordnung (1) nach einem der Ansprüche 1 bis 2,
- wobei die erste Verbindungseinheit (3G) an dem Tabletcomputer (3) angeordnet ist.

4. Anordnung (1) nach einem der Ansprüche 1 bis 3,
- wobei die erste Verbindungseinheit (3G) zumindest einen metallischen Bereich und die zweite Verbindungseinheit (5G) zumindest einen Magneten aufweist und/oder
- wobei die erste Verbindungseinheit (3G) zumindest einen Magneten und die zweite Verbindungseinheit (5G) zumindest einen metallischen Bereich aufweist.

5. Anordnung (1) nach einem der Ansprüche 1 bis 4,
- wobei die Vertiefung (R3) eine Adapterschale (4) aufweist, in welche die Tabletcomputereinheit (3U) formschlüssig aufnehmbar ist.

6. Anordnung (1) nach einem der Ansprüche 1 bis 5,
- wobei die Tabletcomputereinheit (3U) ein erstes Energieübertragungselement (3E) zum Empfang elektrischer Energie für den Tabletcomputer (3) aufweist und/oder
- wobei die Gantry (20) ein mit dem ersten Energieübertragungselement (3E) korrespondierendes zweites Energieübertragungselement(5E) zum Bereitstellen der elektrischen Energie für den Tabletcomputer (3) aufweist.

7. Anordnung (1) nach Anspruch 6,
- wobei die erste Verbindungseinheit (3G) zwei Metallplatten aufweist, wobei das erste Energieübertragungselement (3E) zwischen den zwei Metallplatten (3G1, 3G2) angeordnet ist, und/oder
- wobei die zweite Verbindungseinheit (5G) zwei Magnete (5G1, 5G2) aufweist, welche mit den zwei Metallplatten (3G1, 3G2) korrespondierend angeordnet sind, wobei das zweite Energieübertragungselement (5E) zwischen den zwei Magneten (5G1, 5G2) angeordnet ist.

8. Anordnung (1) nach einem der Ansprüche 6 bis 7,
- wobei das erste Energieübertragungselement (3E) zumindest ein erstes induktives Element aufweist und/oder
- wobei das zweite Energieübertragungselement (5E) zumindest ein zweites induktives Element aufweist.

9. Anordnung (1) nach einem der Ansprüche 1 bis 8, ferner aufweisend eine Befestigungsschale (6), in welche die Tabletcomputereinheit (3U) formschlüssig aufnehmbar ist,
- wobei die Befestigungsschale (6) unabhängig von der Gantry (20) an einer Tragkonstruktion anordenbar ist,
- wobei die Befestigungsschale (6) eine dritte Verbindungseinheit aufweist, welche derart angeordnet ist, dass mittels der ersten Verbindungseinheit (3G) und der dritten Verbindungseinheit eine Verbindung gebildet werden kann, welche einem Entfernen der Tabletcomputereinheit (3U) aus der Befestigungsschale (6) entgegenwirkt.

10. Anordnung (1) nach einem der Ansprüche 1 bis 9, ferner aufweisend die medizinische Bildgebungsvorrichtung (2).

## Claims

1. Arrangement (1), having
- a tablet computer unit (3U) having a tablet computer (3) and a first connection unit (3G),
- a gantry (20) of a medical imaging device (2) having a casing (5) and a second connection unit (5G),
- wherein the casing (5) has a recess (R3) in which the tablet computer unit (3U) can be positively mounted,
- wherein the second connection unit (5G) is arranged in a region of the recess (R3) in such a way that, by means of the first connection unit (3G) and the second connection unit (5G), a connection can be formed, which counteracts removal of the tablet computer unit (3U) from the recess (R3), **characterised in that** the recess (R3) has a first region (R33) for positively mounting the tablet computer (3) and a second region (RG) for receiving the first connection unit (3G),
- wherein the second region (RG) is designed as at least one recess relative to the first region (R33),
- wherein the recess (R3) has a third region (RF), in which positive mounting of the tablet computer (3) is interrupted in such a way that a finger (F) and/or a tool can be applied to the tablet computer (3) in the recess in order to exert a compressive force for removing the tablet computer unit (3U) from the recess (R3).

2. Arrangement (1) according to claim 1,
- wherein the second connection unit (5G) is integrated in the casing (5) and/or arranged on the casing (5).

3. Arrangement (1) according to one of claims 1 to 2,
- wherein the first connection unit (3G) is arranged on the tablet computer (3).

4. Arrangement (1) according to one of claims 1 to 3,
- wherein the first connection unit (3G) has at least one metal region and the second connection unit (5G) has at least one magnet and/or
- wherein the first connection unit (3G) has at least one magnet and the second connection unit (5G) has at least one metal region.

5. Arrangement (1) according to one of claims 1 to 4,
- wherein the recess (R3) has a dock adapter (4) in which the tablet computer unit (3U) can be positively mounted.

6. Arrangement (1) according to one of claims 1 to 5,
- wherein the tablet computer unit (3U) has a first power transmission element (3E) for receiving electrical power for the tablet computer (3) and/or
- wherein the gantry (20) has a second power transmission element (5E) that corresponds with the first power transmission element (3E) for providing the electrical power for the tablet computer (3).

7. Arrangement (1) according to claim 6,
- wherein the first connection unit (3G) has two metal plates, wherein the first power transmission element (3E) is arranged between the two metal plates (3G1, 3G2), and/or
- wherein the second connection unit (5G) has two magnets (5G1, 5G2), which are arranged to correspond with the two metal plates (3G1, 3G2), wherein the second power transmission element (5E) is arranged between the two magnets (5G1, 5G2).

8. Arrangement (1) according to one of claims 6 to 7,
- wherein the first power transmission element (3E) has at least one first inductive element and/or
- wherein the second power transmission element (5E) has at least one second inductive element.

9. Arrangement (1) according to one of claims 1 to 8, also having a mounting tray (6), in which the tablet computer unit (3U) can be positively mounted,
- wherein the mounting tray (6) can be arranged on a support construction independently of the gantry (20),
- wherein the mounting tray (6) has a third connection unit, which is arranged in such a way that, by means of the first connection unit (3G) and the third connection unit, a connection can be formed, which counteracts removal of the tablet computer unit (3U) from the mounting tray (6).

10. Arrangement (1) according to one of claims 1 to 9, also having the medical imaging device (2).

## Revendications

1. Système (1), comportant
- une unité (3U) d'ordinateur à tablette, ayant un ordinateur (3) à tablette et une première unité (3G) de liaison,
- un portique (20) d'une installation (2) d'imagerie médicale ayant un habillage (5) et une deuxième unité (5G) de liaison,
- dans lequel l'habillage (5) a une cavité (R3), dans laquelle l'unité (3U) d'ordinateur à tablette peut être reçue à complémentarité de forme,
- dans lequel la deuxième unité (5G) de liaison est disposée dans une partie de la cavité (R3), de manière à pouvoir former, au moyen de la première unité (3G) de liaison et de la deuxième unité (5G) de liaison, une liaison, qui s'oppose au retrait de l'unité (3U) d'ordinateur à tablette de la cavité (R3),
**caractérisé en ce que** la cavité (R3) a une première partie (R33) de réception à complémentarité de forme de l'ordinateur (3) à tablette et une deuxième partie (RG) de réception de la première unité (3G) de liaison,
- dans lequel la deuxième partie (RG) est constituée comme au moins une cavité par rapport à la première partie (R33),
- dans lequel la cavité (R3) a une troisième partie (RF), dans laquelle la réception à complémentarité de forme de l'ordinateur (3) à tablette est interrompue, de manière à pouvoir mettre dans la cavité un doigt (F) et/ou un outil sur l'ordinateur (3) à tablette, afin d'appliquer une force de pression pour retirer l'unité (3U) d'ordinateur à tablette de la cavité (R3).

2. Système (1) suivant la revendication 1,
- dans lequel la deuxième unité (5G) de liaison est intégrée à l'habillage (5) et/ou est disposée sur l'habillage (5).

3. Système (1) suivant l'une des revendications 1 à 2,
- dans lequel la première unité (3G) de liaison est disposée sur l'ordinateur (3) à tablette.

4. Système (1) suivant l'une de revendications 1 à 3,
- dans lequel la première unité (3G) de liaison a au moins une partie métallique et la deuxième unité (5G) de liaison a au moins un aimant et/ou
- dans lequel la première unité (3G) de liaison a au moins un aimant et la deuxième unité (5G) de liaison a au moins une partie métallique.

5. Système (1) suivant l'une des revendications 1 à 4,
- dans lequel la cavité (R3) comporte une coquille (4) d'adaptateur, dans laquelle l'unité (3U) d'ordinateur à tablette peut être reçue à complémentarité de forme.

6. Système (1) suivant l'une des revendications 1 à 5,
- dans lequel l'unité (3U) d'ordinateur à tablette a un premier élément (3E) de transport d'énergie pour recevoir de l'énergie électrique pour l'ordinateur (3) à tablette et/ou
- dans lequel le portique (20) a un deuxième élément (5E) de transport d'énergie correspondant au premier élément (3E) de transport d'énergie pour mettre de l'énergie électrique à disposition de l'ordinateur (3) à tablette.

7. Système (1) suivant la revendication 6,
- dans lequel la première unité (3G) de liaison a deux plaques métalliques, le premier élément (3E) de transport d'énergie étant disposé entre les deux plaques (3G1, 3G2) métalliques, et/ou
- dans lequel la deuxième unité (5G) de liaison a deux aimants (5G1, 5G2), qui sont disposés de manière correspondante avec les deux plaques (3G1, 3G2) métalliques, le deuxième élément (5E) de transport d'énergie étant disposé entre les deux aimants (5G1, 5G2).

8. Système (1) suivant l'une des revendications 6 à 7,
- dans lequel le premier élément (3E) de transport d'énergie a au moins un premier élément inductif et/ou
- dans lequel le deuxième élément (5E) de transport d'énergie a au moins un deuxième élément inductif.

9. Système (1) suivant l'une des revendications 1 à 8, comportant, en outre, une coquille (6) de fixation, dans laquelle l'unité (3U) d'ordinateur à tablette peut être reçue à complémentarité de forme,
- dans lequel la coquille (6) de fixation peut être disposée sur une construction porteuse, indépendamment du portique (20),
- dans lequel la coquille (6) de fixation a une troisième unité de liaison, qui est disposée de manière à pouvoir, au moyen de la première unité (3G) de liaison et de la troisième unité de liaison, former une liaison, qui s'oppose à un retrait de l'unité (3U) d'ordinateur à tablette de la coquille (6) de fixation.

10. Système (1) suivant l'une des revendications 1 à 9, comportant, en outre, l'installation (2) d'imagerie médicale.
